# EUROPÄISCHE PATENTSCHRIFT

(11) **EP 2 008 638 B1**
(45) Veröffentlichungstag und Bekanntmachung des Hinweises auf die Patenterteilung: **22.02.2012**
(21) Anmeldenummer: 08156454.4
(22) Anmeldetag: 19.05.2008
(51) Int. Cl.: A61K 8/06, A61K 8/22, A61Q 15/00

(54) **Verwendung von Sauerstoff-hältigen kosmetischen oder dermatologischen Zubereitungen als Deodorant und/oder Antitranspirant**
Use of cosmetic preparations comprising oxygen as deodorants and/or antiperspirants
Utilisation de préparations cosmétiques comprenant de l'oxygène comme produits déodorants et/ou antitranspirants

(30) Priorität: 14.06.2007 DE 102007028028
(43) Veröffentlichungstag der Anmeldung: 31.12.2008
(73) Patentinhaber: Beiersdorf AG, 20245 Hamburg (DE)
(72) Erfinder: Mummert, Christopher, Dr., 29553 Bienenbüttel (DE); Biel, Stefan, Dr., 21077 Hamburg (DE); Kuhlmann, Maike, Dr., 22529 Hamburg (DE); Kolbe, Ludger, Dr., 21255 Dohren (DE); Wöller, Karl-Heinz, 20257 Hamburg (DE); Achterberg, Volker, Dr., 22159 Hamburg (DE); Meyer, Maren, 22459 Hamburg (DE); Schreiber, Jörg, Dr., 22087 Hamburg (DE); Balcke, Isabel, 22337 Hamburg (DE); Hahn, Ingo, Dr., 25421 Pinneberg (DE); Bleckmann, Andreas, 22926 Ahrensburg (DE); Blatt, Thomas, Dr., 22880 Wedel (DE); Meier-Zimmerer, Cornelia, Dr., 90425 Nürnberg (DE); Schwengler, Helge, 21244 Buchholz (DE); Rathsack, Jessica, 21614 Buxtehude (DE); Lenz, Holger, 4052 Basel (CH)

(56) Entgegenhaltungen:
- WO-A-96/33695
- WO-A-03/022238
- WO-A-2005/027869
- DE-A1-102005 011 457

## Beschreibung

Die Erfindung umfasst die Verwendung einer kosmetische Zubereitung auf Basis einer Emulsion umfassend molekularen Sauerstoff. Die Stabilität der mit Sauerstoff beladenen Zubereitungen und die Sauerstoffverfügbarkeit aus den Zubereitungen ist gegenüber bekannten sauerstoffhaltigen Zubereitungen verbessert.

Gas-haltige kosmetische Zubereitungen sind an sich bekannt und bereits in zahlreichen Patenten beschrieben worden. Als Gase werden beispielsweise Sauerstoff, fluorierte Gase, Kohlendioxid, Luft, Stickstoff und dergleichen verwendet.

Bereits seit langem ist die essentielle Bedeutung von Sauerstoff für die Wundheilung bekannt (siehe z.B. in Hunt TK, Zederfeldt B, Goldstick TK. Oxygen supply in healing tissue. Am J Surg 1969; 123:247-252).
Viele Prozesse der Wundheilung sind auf eine ausreichende Sauerstoffversorgung angewiesen. Die Rolle von Sauerstoff auf die Proliferation von Fibroblasten und Keratinozyten ist beschrieben. Sauerstoff gilt als Substrat für Enzyme, die an der Kollagensynthese beteiligt sind, hat Effekte auf Zytokine und Wachstumsfaktoren sowie die Angiogenese und spielt eine Rolle bei der Abwehr von Bakterien. Sauerstoff ist nicht nur für die zelluläre Atmung und als Substrat für bestimmte Enzyme wichtig, für die aus dem Sauerstoff gebildeten reaktiven Sauerstoffspezies ROS wird ferner eine entscheidende Rolle als Signalmoleküle in der Wundheilungskaskade diskutiert.
Experimentelle Untersuchungen, in der Literatur beschrieben, belegen die positiven Eigenschaften eines kurzzeitigen Überangebots an Sauerstoff auf dermale Keratinozyten und dermale Fibroblasten sowie eine Konzentrationszunahme von Prokollagen in vitro.
Die Angaben des Sauerstoffpartialdrucks in dermalen Wunden gehen von Werten unterhalb von 10 mm Hg bzw. 20 mm Hg im Zentrum chronischer Wunden und 60 mmHg in den Randbereichen der Wunde aus (siehe z.B. Tandara AA, Mustoe TA. Oxygen in wound healing - more than a nutrient. World J Surg 2004; 28:294-300).
Aufgrund von Untersuchungen an chronischen Wunden geht man davon aus, dass ein transkutaner Sauerstoffpartialdruck von weniger als 40 mm Hg Wundheilungsstörungen begünstigt (siehe z.B. Dissemond J. Körber A. Jansen T. Schneider LA. The role of oxygen in the treatment of leg ulcers. Zeitschrift für Wundheilung 2005; 6: 252-256)

Der Einsatz von Sauerstoff ist bereits seit Jahren gängige Praxis in der Wundheilung. So gilt die hyperbare Sauerstofftherapie (HOB) bei der Behandlung von chronischen Problemwunden, z.B. bei diabetischen Ulcera, als etabliert. Für die HOB konnte gezeigt werden, dass durch eine kurzzeitige intermittierende Erhöhung des Sauerstoffpartialdruckes im Blut die Wiederherstellung von physiologischen Gewebesauerstoffwerten im hypoxischen Areal erreicht werden kann. In speziellen hyperbaren Druckkammern sind die Patienten kurzzeitig 100% reinem Sauerstoff bei einem Umgebungsdruck von 1.5 - 3.0 atm ausgesetzt, so dass es zu einen Anstieg des physikalisch gelösten Sauerstoff im Blut und zu einem erhöhten Perfusionsdruck kommt. Eingesetzt wird die HOB vor allem beim diabetischen Fusssyndrom.
Auch die Anwendung von topischen Applikationsformen von Sauerstoff und der positive Effekt auf die Wundheilung sind beschrieben. So konnte z.B. bei topischer Sauerstoffbehandlung an dermalen Schnittwunden im Tierversuch ein Anstieg des pO2 im Gewebe gezeigt werden, wiederholte Anwendung führte zu einer verbesserten Angiogenese und Gewebsperfusion und einer beschleunigten Wundheilung (s. z.B. Fries RB, Wallace WA, Roy S, Kuppusamy P, Bergdall V, Gordillo GM, Melvin WS, Sen CK. Dermal excisional wound healing in pigs following treatment with topically applied pure oxygen. Mutat Res. 2005 Aug 13.)
In den letzten Jahren wurden verschiedene Produkte vorgestellt, die die topische Applikation von Sauerstoff ermöglichen und einen Einfluss auf den Verlauf der Wundheilung zeigen. So konnte für die Applikation 100% Sauerstoff lokal in der Wundregion mittels einer speziellen topischen Kammer ein positiver Effekt in der Behandlung von akuten und chronischen Wunden gezeigt werden (s. z.B. Kalliainen LK, Gordillo GM, Schlanger R, Sen CK. Topical oxygen as an adjunct to wound healing: a clinical case series. Pathophysiology. 2003 Jan;9(2):81-87.).

Ausgangspunkt der Entwicklungen zur hyperbaren Sauerstofftherapie waren stationäre Druckkammern analog solcher zur Behandlung von Taucherkrankheit, bei der der Patient insgesamt einer Sauerstoffatmosphäre ausgesetzt wurde. In der Weiterentwicklung dieser Therapie kamen kleinere Systeme zur Anwendung, bei denen nur das betroffenen Körperareal von einer gasdichten Umhüllung eingeschlossen und mit gasförmigem Sauerstoff behandelt wurde.

DE 69631587 T2 beschreibt ein Wundverbandstück in pflaster- bzw. bandagenform zur topischen Behandlung von Hautwunden, bei dem der Sauerstoff durch elektrochemische

Prozesse innerhalb der Bandage erzeugt wird. Aufgrund der Vielzahl der dafür notwendigen Einzelkomponenten (Pflastermaterialien, Chemikalien, Energiequellen, Regelelemente etc.) muss ein solches Wundverbandstück entsprechend komplex aufgebaut sein, was für den Anwender mit geringerem Applikations- und Tragekomfort einher geht.

WO 03105797 A1 beschreibt Mikroemulsionen mit binärer Phasen- und Wirkstoffdifferenzierbarkeit, deren Herstellung und deren Verwendung, insbesondere zur topischen Sauerstoffversorgung. Die in dieser Schrift vorgestellten Darreichungsformen benötigen biologische Sauerstoffträger, ausgewählt aus nativem, modifiziertem oder unmodifiziertem Hämoglobin, Myoglobin oder Mischungen hiervon. Freier oder in den Mikroemulsionen gelöster molekularer Sauerstoff kommt nicht zur Anwendung.

US 2005/0244354 A1 beschreibt Personal Care Produkte für kosmetische oder medizinische Anwendungen jedweder Art bis hin zu mit Sauerstoff angereichertem Trinkwasser, wobei diese Produkte aus einem Substrat und flüssigem Sauerstoff bestehen. Als zusätzliche Sauerstoffdonatoren werden Ozon und Wasserstoffperoxyd eingearbeitet.

Eine Möglichkeit zur Stabilisierung von Gasen in kosmetischen oder dermatologischen Zubereitungen besteht z. B. darin, eine O/W-Emulsion herzustellen, welche anschließend mit Gas beaufschlagt wird. Derartige Formulierungen sind auch als Mousse bekannt, wie beispielsweise in WO 2002-074258-A1 beschrieben. Nachteilig an diesen Zubereitungen des Standes der Technik ist, dass das Gas nur durch den Einsatz von bestimmten Emulgatoren stabilisiert werden kann. Ferner nachteilig ist, dass das Gas bei der topischen Applikation oder bereits bei Lagerung bei 40 °C oder mehr (beispielsweise im Auto oder am Strand) in die Atmosphäre entweichen kann. Ferner ist die Beladungskapazität derartiger Zubereitungen meistens eher gering, so dass sich physiologische Effekte nach der Applikation nicht einstellen.

Aus dem Stand der Technik sind des weiteren kosmetische Zubereitungen auf Basis von O/W-Emulsionen bekannt, die Sauerstoff enthalten.
Die WO 02/05754 A2 beschreibt extern applizierbare Zubereitungen, die einen Sauerstoffträger enthalten, der in einer lipoiden Emulsion molekulardispers eingearbeitet ist, sowie deren Verwendung zur externen Behandlung/Prävention von Sauerstoffmangelzuständen der Haut.

Die WO 02/05754 A2 offenbart O/W-Emulsionen, die einen Sauerstofftransport durch die Lipidschicht der Haut adäquat gewährleisten sollen.

In der US 5851544 wird beschrieben, dass Hautpflegezubereitungen, welche das Hautsauerstoffniveau erhöhen, wünschenswert seien, da der Sauerstoffgehalt in der Haut niedriger sei als in anderen Körperregionen und darüber hinaus mit zunehmendem Alter abnehme. Aufgabe der US 5851544 ist es, den Sauerstoffgehalt der Haut langfristig zu steigern bzw. Sauerstoffmangelzustände in der Haut zu beheben.

In der DE 10333710 A1 werden kosmetische, dermatologische oder pharmazeutische Zubereitungen auf Basis von Lipiden und/oder Lipid/Wachs-Gemischen, welche ein Gas bzw. ein Gasgemisch enthalten, beschrieben.

Die DE 10342449 A1, DE 102005011498 A1, DE 102005011457 A1 und DE 102206011459 A1 offenbaren kosmetische Zubereitungen enthaltend molekularen Sauerstoff. Auch hierin werden O/W-Emulsionen beschrieben. Die Herstellung der sauerstoffhaltigen O/W-Emulsionen erfolgt indem eine Vordispergierung des anorganischen Gelbildners und Quellung des Hydrokolloides unter Rühren in der Wasserphase stattfindet. Eine Vereinigung der auf 75 °C aufgeheizten Fettphase mit der auf 70 °C aufgeheizten Wasserphase und Zugabe der partikulären hydrophoben, hydrophobisierten Festkörpersubstanzen erfolgt unter Rühren. Es erfolgt dann Homogenisierung bei 65 °C und 45 min Rühren unter Vakuum und Kühlung. Die Zugabe der Additive erfolgt bei 30 °C, die Homogenisierung bei 27 °C. Anschließend, also bei gekühlten Temperaturen, wird die Begasung der Emulsion bei 7-8 bar mit Sauerstoff durchgeführt.

WO 2005/027869 A1, DE 102005011457 A1, WO 96/33695 A1 offenbaren sauerstoffhaltige Zubereitungen auf Emulsionsbasis.
WO 2005/027869 A1 beschreibt die Herstellung einer solchen Zubereitung bei einer Temperatur von 65°C, wobei deren Begasung mit Sauerstoff unter Kühlung auf 30°C erfolgt.

Die Herstellung sauerstoffhaltiger kosmetischer Zubereitungen und insbesondere der Schritt der Sauerstoffzufuhr erfolgt in allen aufgeführten Druckschriften des Standes der Technik dabei unter Kühlung, bei Raumtemperatur bzw. bei maximal 30°C.

Nachteilig an den bekannten Zubereitungen ist, dass nach Einarbeitung des molekularen Sauerstoffs, die Zubereitungen nicht stabil lagerfähig sind, eine geringe Sauerstoffbeladungskapazität aufweisen und eine geringe bis unakzeptable Sauerstoffverfügbarkeit von weniger als 15 Vol%, mitunter weniger als 5 Vol.% Sauerstoff aufweisen.

Nachteilig ist weiterhin, dass es sich häufig um O/W-Emulsionen handelt in denen der Sauerstoff enthalten ist. Bekannt ist jedoch dass einige W/O-Emulgatoren für die Haut und insbesondere Wunden im allgemeinen besser verträglich als einige O/W-Emulgatoren sind.

Wünschenswert ist es daher eine kosmetische Zubereitung bereit zu stellen, die molekularen Sauerstoff enthält und die eine für Kosmetika ausreichende Lagerfähigkeit aufweist. Ebenso ist es wünschenswert ein Verfahren zur Herstellung sauerstoffhaltiger Emulsionen zur Verfügung zu haben, mit dem eine kosmetisch anwendbare und lagerfähige Zubereitung herstellbar ist.
Wünschenswert ist es darüber hinaus eine hautvertägliche und eine auch für die Wundheilung anwendbare Zubereitung bereit zu stellen.

Unter kosmetischer Hautpflege ist in erster Linie zu verstehen, dass die natürliche Funktion der Haut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Wünschenswert ist es auch Produkte zur Pflege der auf natürliche Weise gealterten Haut, sowie zur Behandlung der Folgeschäden der Lichtalterung zur Verfügung zu stellen.
Produkte zur Pflege gealterter Haut sind an sich bekannt. Sie enthalten z. B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Produkte zum Schutz vor schädlichen Oxidationsprozessen in der Haut sind an sich bekannt. Auch Produkte, welche die Sauerstoff-Versorgung der Haut steigern sollen, sind bereits im Stand der Technik beschrieben. Allerdings konnte der Stand der Technik nicht den Weg zur vorliegenden Erfindung weisen.

Überraschenderweise wurde nun festgestellt, dass in eine kosmetische Zubereitung, insbesondere auf Basis einer W/O-Emulsion, molekularer Sauerstoff stabil und lagerfähig eingebunden werden kann.
Es ist erfindungsgemäß möglich Zubereitungen mit einer Sauerstoffverfügbarkeit von mehr als 10 Vol. % bereit zu stellen

Die erfindungsgemäße Verwendung einer kosmetischen Zubereitung auf Basis einer Emulsion als Deodorant und/oder Antitranspirant, insbesondere einer W/O-Emulsion, umfassend molekularen Sauerstoff ist dadurch charakterisiert, dass sie erhältlich ist durch übliches Mischen und Herstellen einer Emulsion und anschließendem Begasen der Zubereitung mit gasförmigen Sauerstoff und/oder sauerstoffhaltigen Gasen. Wesentlich dabei ist, dass die Zubereitung während der Begasung eine Temperatur von mindestens 40 °C aufweist, insbesondere mehr als 40°C, besonders vorteilhaft mehr als 45 °C.

Bei den aus dem Stand der Technik bekannten üblichen Sauerstoffbegasungen konnten keine hinreichende Menge (> 5 Vol-%) Sauerstoff lagerstabil in die Emulsion eingetragen werden. Zudem verliefen diese Begasungen immer bei niedrigen Temparaturen, maximal bei Raumtemperatur.

Die erfindungsgemäß hergestellten Zubereitungen unterscheiden sich von den sauerstoffhaltigen Zubereitungen dadurch, dass der Sauerstoff länger und in größerem Umfang in der Zubereitung zur Verfügung steht. Ein weiterer Unterschied ist die Bereitstellung als sauerstoffhaltige W/O-Emulsion, so dass es besonders vorteilhaft im Sinne der vorliegenden Erfindung ist, Sauerstoff in Form von W/O-Emulsion basierenden Zubereitungen auf die Haut aufzubringen.

Der Sauerstoffvolumenanteil einer erfindungsgemäßen Zubereitung bei 21°C. wobei dies nicht die Zugabetemperatur ist, kann vorteilhaft auf einen Anteil von bis zu 40 Vol.% erhöht werden und er liegt bevorzugt zwischen 5 und 20 Vol.%, insbesondere mehr als 10 Vol.%. Die Sauerstofffreisetzung und -anteil kann wie folgt bestimmt werden.
Nach Einwaage der zu vermessenden Probe in eine Headspace-Ampulle wird diese verschlossen und der Gasraum mit Stickstoff gespült. Um den Sauerstoff aus der Probe zu treiben wird die Ampulle bei 80°C/über Nacht äquilibriert.
Durch Injektion aus dem Gasraum über der Probe erfolgt die Analyse per Gaschromatographie mittels Wärmeleitfähigkeitsdektion.
Messgrundlage ist das prozentuale Verhältnis der Peakflächen von Sauerstoff und Stickstoff in dem Gasraum über der Emulsion. Hieraus folgt die Berechnung von ml O₂ /100g Probe. Dieser Wert wird über das Molvolumen von Sauerstoff unter Normalbedingungen in mg O₂ /kg Probe umgerechnet und kann abschließend auf Vol.% bezogen auf das Volumen der Zubereitung umgerechnet werden.

Nachfolgend wird beispielhaft die Herstellung einer sauerstoffhaltigen W/O-Emulsion beschrieben.
Die Herstellung der sauerstoffhaltigen W/O-Emulsion erfolgt, in dem in die auf 80°C erwärmte Fettphase die in der Formulierung enthaltenen Feststoffe eindispergiert werden. Die so hergestellte Suspension wird mit der auf 80°C erwärmten Wasserphase vereinigt. Die Emulsion wird dann unter Rühren abgekühlt. Die Zugabe von weiteren Stoffen oder weiteren Wirkstoffen erfolgt kurz vor der Heißhomogenisierung bei 65°C. Über einen Zeitraum von ca. 40-50 min wird die Emulsion unter Rühren auf 30°C abgekühlt. Kurz vor der Kalthomogenisierung bei 30°C werden ggf. temperaturempfindliche Stoffe wie Parfüms etc. zugegeben.
Die so erhaltene cremeartige Emulsion wird nun in einem zweiten, erfindungswesentlichen, Produktionsschritt mit reinem Sauerstoff versetzt. Alternativ kann auch mit sauerstoffhaltigen Gasen, wie Luft, gearbeitet werden.
Hierzu wird die erhaltene Emulsion auf eine Temperatur von mindestens 40°C erwärmt. Die Maximaltemperatur liegt vorteilhaft bei etwa 50°C. Anschließend wird die erwärmte Emulsion einem dynamischen Schaumgenerator (z.B. Top-Mix von Hansa-Industrie-Mixer) zugeführt. Mit Hilfe des dynamischen Schaumgenerators wird der Emulsion eine definierte Menge Sauerstoff (vorteilhaft etwa 10 bis 20 Vol.%) in Form von Gasbläschen zudosiert. Der im Schaumgenerator enthaltene Rotor-Stator-Mixkopf sorgt bei einer Drehzahl von 100 bis 600 min⁻¹ und einem Systemdruck von 1-4 bar für eine Feinstverteilung der Gasbläschen. Nach der Vearbeitung im Schaumgenerator kann die Emulsion gekühlt werden, so dass der entstehende Schaum den Mixkopf mit einer Temperatur von ca. 35 °C verlässt.
Entscheidener Schritt ist, dass der im Schaumgenerator zugeführte Sauerstoff bei einer Temperatur von 40°C und mehr zugegeben wird. Dadurch wird überraschenderweise ein hoher Sauerstoffgehalt in der dann fertigen Zubereitung erhalten und dieser O₂-Gehalt kann dann später wieder freigesetzt werden, wenn die Zubereitung beispielweise auf die Haut appliziert wird.
Dies ist insbeondere überraschend, da eine Begasung bei höherer Temperatur aus themodynamischen Gründen eigentlich eine geringere Gasbeladung erwarten lässt.

Die kosmetischen Zubereitungen gemäß der Erfindung können kosmetische Hilfsstoffe enthalten, wie sie üblicherweise in solchen Zubereitungen verwendet werden, z. B. Emulgatoren, Pigmente, Konservierungsmittel, Konservierungshelfer, Komplexbildner, Bakterizide, Parfüme, Substanzen zum Steigern des Schäumens, Farbstoffe, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Weitere bevorzugte Ausführungsform der erfindungsgemäßen Zubereitungen umfasst lediglich eine Emulsion mit nur einem Emulgator, insbesondere W/O-Emulsionen mit nur einem W/O-Emulgator.

Diese Zubereitungen, umfassend nur einen Emulgator und insbeondere Polyglyceryl-3 Diisostearat, lassen sich in der Wärme besonders gut mit Sauerstoff begasen.

Es ist daher weiter vorteilhaft als Emulgator Polyglyceryl-3 Diisostearate in der erfindungsgemäßen Zubereitung einzusetzen, dies insbesondere als alleinigen Emulgator. Vorteilhaft wird Polyglyceryl-3 Diisostearate zu einem Anteil von 0,5 bis 10 Gew.%, bevorzugt 1,0 bis 5 Gew.%, jeweils bezogen auf die Gesamtmasse der Zubereitung, eingesetzt.

Die erfindungsgemäße sauerstoffhaltige Zubereitung auf Basis einer W/O-Emulsion umfasst daher bevorzugt Polyglyceryl-3 Diisostearat als Emulgator.

Als weitere oder als alleinige Emulgatoren sind vorteilhaft Cetyl Dimethicone Copolyol (ABIL EM 90), PEG-30 Dipolyhydroxystearate (Arlacel P135), Polyglyceyldipolyhydroxystearate (PGPH), Polygylceryl-3 Diisostearat (Emulgator IGT) und/oder Eucerit zu nennen.

Ebenso überaschend zeigt sich, dass sich in die erfindungsgemäßen Zubereitungen ein hoher Pigmentanteil einarbeiten lässt.
Die Vorteile eines hohen Pigmentanteils liegen in den verursachenden antibakteriellen Eigenschaften und in der Erzielung eines physikalischen Schutzes auf der Haut, der wasserabweisend bzw. feuchtigkeitsabweisend ist.
Dies scheiterte häufig an dem Umstand nur geringe Mengen an Pigmenten in emulsionsbsierenden kosmetischen Zubereitungen einbauen zu können ohne Einbussen hinsichtlich Stabilität und vor allem Sensorik hin nehmen zu müssen.

Als bevorzugte Pigmente sind ZnO, Talkum, TiO₂, FeO und deren Kombinationen zu nennen.
Bekannt ist der Zusatz beispielweise von Zinkoxiden, die zum Wundschutz, Linderung von Hautrötungen und zur Wundheilung zugesetzt werden können.
Erfindungsgemäß wird der Zusatz von ZnO auf einen Anteil von mindestens 5 Gew.%, insbesondere mehr als 5 Gew.%, erhöht.

Bevorzugt werden nicht-"gecoatete" Pigmente eingesetzt. Gecoatet Pigmente werden häufig als Lichtschutzfilterpigmente benannt bzw. eingesetzt, so dass auf deren Einsatz verzichtet werden kann.
Der Pigmentanteil liegt bevorzugt bei mehr als 5 Gew.%, insbesondere bei etwa 10 Gew.% und mehr, bevorzugt maximal bei etwa 60 Gew.% und idealerweise im Bereich von 15 bis 45 Gew.%.
Bevorzugt wird auf den Zusatz von chemischen Lichtfilter bzw. gecoateten Pigmenten gänzlich verzichtet. Vorteilhaft umfasst die efindungsgemäßen Zubereitungen keine Lichtschutzfiltersubstanzen.

Bevorzugt ist daher eine W/O-Emulsion umfassend mehr als 10 Vol.% Sauerstoff und mehr als 5 Gew.% Pigmente, insbesondere nicht-gecoatete Pigmente.
Vorteilhaft ist dann noch, wenn nur ein W/O-Emulgator und insbeondere Polyglyceryl-3 Diisostearat, enthalten ist.

Eine Zubereitung umfassend Sauerstoff, einen hohen Anteil an nicht-gecoateten Pigmenten und/oder Polyglyceryl-3 Diisostearat als Emulgator, unabhängig wie der Sauerstoff in die Zubereitung eingebracht wird, ist somit eine ebenfalls erfindungemäße Zubereitung.

In diesen Zubereitungen hält sich der Sauerstoffgehalt besondere lang und stabil im Bereich von mehr als 10 Vol.%, insbesondere mehr als 15 Vol%.

Die kosmetischen Zubereitungen gemäß der Erfindung können vorteilhaft in Form von schäumbaren Zubereitungen vorliegen, welche beispielsweise aus Aerosolbehältern entnommen und dabei aufgeschäumt werden. Erfindungsgemäß vorteilhafte Aerosolbehälter sind Sprühvorrichtungen mit einer Füllung aus den flüssigen bzw. breiartigen Stoffen, die unter dem Druck eines Treibmittels stehen (Druckgas- oder Aerosolpackungen). Derartige Behälter können mit Ventilen sehr unterschiedlicher und bekannter Bauart ausgestattet sein, die die Entnahme des Inhalts als Schaum ermöglichen.

Zur Anwendung werden die kosmetischen Zubereitungen in der für Kosmetika üblichen Weise auf die Haut und/oder die Haare in ausreichender Menge aufgebracht.

Die erfindungsgemäße Verwendung ist als Deodorant und/oder Antitranspirant.

Die bakterielle Zersetzung (gram positive anaerobe Kokken) von axillärem Schweiß führt zu der Entstehung von Stoffwechselprodukten, die einen extrem unangnehmen Gruch besitzen können. Um diesen Geruch zu vermindern oder verhindern werden desodorierende Wirkstoffe eingesetzt, die die gesamte Achselflora abtöten. Dabei werden auch Keime der Hautflora abgetötet, die nicht zum Achselgruch beitragen. Dies hat wiederum Hautschäden oder Irritationen zur Folge.

Überaschenderweise zeigte sich, dass die erfindungsgemäße Zubereitung durch den Sauerstoffgehalt zur Erhöhung der Hautzellregeneration in der Achsel beiträgt. Daneben zeigte sich aber ebenso überraschend, dass auch ein Teil der mikrobiellen Hautflora durch den freigesetzten Sauerstoff abgetötet wird. Dabei wurden aber insbesondere nur die gram positiven anaeroben Kokken und die mikroaerotoleranten Mikroorganismen, deren Stoffwechselprodukte besonders zur Geruchsentstehung beitragen, abgetötet.
Durch die erfindungsgemäße Zubereitung ist somit ein gezieltes Abtöten relevanter Keime möglich geworden ohne dass die gesamte Hautflora in Mitleidenschaft gezogen wird.
Es wurden Untersuchungen zum Einfluss von Sauerstoff auf achselrelevante Bakterien durchgeführt (s. Abbildung). Es wurden dabei die Keime Staphylococcus epidermidis (S. epidermidis), Corynebacterium (C. jeikeium), Peptostreptococcus (P. harei und A. octavius) und deren Reduktion mit und ohne Sauerstoff ermittelt. Es zeigt sich, dass durch Sauerstoff nur ausgewählte Bakterien-Spezies abgetötet werden (P. harei und A. octavius) andererseits aber auch die Hautzellregeneration angeregt wird (Staphylococcus epidermidis).
Vorteilhaft ist, dass die erfindungsgemäße Zubereitung als Deodorant bzw. Antitranspirant verwendet werden kann und dabei insbesondere auf Antitranspirantwirkstoffe, wie Aluminiumchlorohydrate, dann verzichten werden kann.

Zusätzlich zu Sauerstoff können erfindungsgemäße Zubereitungen weitere pharmazeutische, kosmetische oder dermatologische Wirkstoffe enthalten. Z.B. Kamillenextrakte, Bisabolol, Dexpanthenol, Aloe vera, Vitamine, Hyaluronsäure, ätherische Öle, essentielle Fetssäuren, feuchthaltende Substanzen, Proteine und Proteinderivate, Polysaccharide, Pflanzenextrakte, Aminosäuren, Peptide etc. enthalten.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiel 1: W/O-Emulsion

| **Bestandteil** | **Gew.%** |
|---|---|
| Cera Microcristallina + Paraffinum Liquidum | 42,5 |
| Zinc Oxide | 20,0 |
| Lanolin | 15,0 |
| Talc | 10,0 |
| Aqua | 7,4 |
| Polyglyceryl-3 Diisostearate | 1,5 |
| Triticum Vulgare | 1,0 |
| Maris Sal | 1,0 |
| Panthenol | 0,5 |
| Parfum | 0,4 |
| Phenoxyethanol | 0,36 |
| Methylparaben | 0,3 |
| Propylparaben | 0,04 |
| Summe | 100,0 |
| Sauerstoffgehalt (Vol.%), initial | 11,97 - 13,05 |
| nach 24 und 48 h: | 11,87 - 13,00 |

### Beipsiel 2: Wundheilsalbe (nicht erfindungsgemäß)

| **Bestandteil** | **Gew.%** |
|---|---|
| Cera Microcristallina + Paraffinum Liquidum | 5,0 |
| Cera Microcristallina | 3,0 |
| Lanolin Alcohol | 0,7 |
| Paraffinum Liquidum | 13,6 |
| Isopropyl Palmitate | 2,0 |
| Polyglyceryl-3 Diisostearate | 2,5 |
| Aluminum Stearate | 0,4 |
| Magnesium Stearate | 0,01 |
| Panthenol | 6,43 |
| Phenoxyethanol | 0,5 |
| Sodium Citrate | 0,65 |
| Potassium Sorbate | 0,2 |
| Magnesium Sulfate | 0,7 |
| Glycerin | 2,6 |
| Citric Acid | 0,35 |
| Aqua | ad |

Die Wundheilsalbe wird separat nach den üblichen Verfahren hergestellt und im Hansamischer mit O₂ bei T = 42°C beaufschlagt. Der Gehalt an O₂ im Endprodukt Wundheilsalbe dieser Rezeptur beträgt 118 mg/kg, was einem max. Volumenanteil von 8,9 Vol.% bei 21 °C, berechnet als Schaum ohne gelösten Sauerstoff, entspricht.

### Beispiel 3: W/O-Emulsion

| **Bestandteil** | **Gew.%** |
|---|---|
| Cera Microcristallina + Paraffinum Liquidum | 41,7 |
| Zinc Oxide | 20,4 |
| Lanolin | 15,2 |
| Talc | 10,2 |
| Aqua | 7,4 |
| Polyglyceryl-3 Diisostearate | 1,4 |
| Triticum Vulgare | 1,0 |
| Maris Sal | 1,0 |
| Panthenol | 0,5 |
| Parfum | 0,5 |
| Phenoxyethanol | 0,36 |
| Methylparaben | 0,3 |
| Propylparaben | 0,04 |
| Summe | 100,0 |
| Sauerstoffgehalt (Vol.%), initial | 15,6 - 16,0 |
| nach 24 und 48 h: | 15,6 - 15,9 |

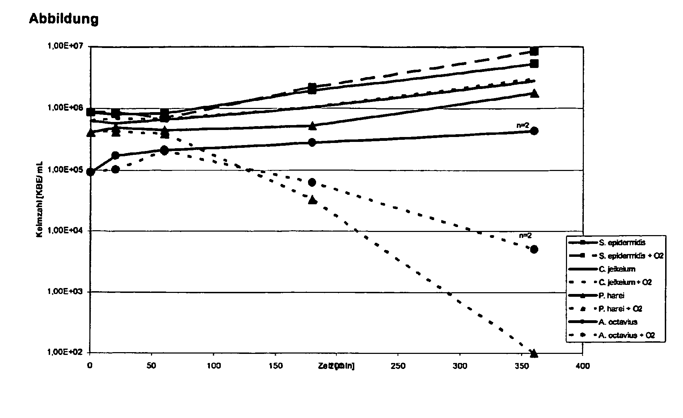

## Patentansprüche

1. Verwendung einer kosmetischen Zubereitung auf Basis einer Emulsion umfassend molekularen Sauerstoff erhältlich durch Mischen der Emulsionsbestandteile und anschließendem Begasen der Zubereitung mit gasförmigen Sauerstoff und/oder sauerstoffhaltigen Gasen, **dadurch gekennzeichnet, dass** die Zubereitung während der Begasung eine Temperatur von mindestens 40°C aufweist, als Deodorant und/oder Antitranspirant.

2. Verwendung der Zubereitung nach Anspruch 1 umfassend einen Volumenanteil an Sauerstoff bei 21°C von mehr als 10 Vol.%, bezogen auf das Gesamtvolumen der Zubereitung.

3. Verwendung der Zubereitung nach einem der vorstehenden Ansprüche, **dadurch gekennzeichnet, dass** die Zubereitung eine W/O-Emulsion ist.

4. Verwendung der Zubereitung nach einem der vorstehenden Ansprüche, **dadurch gekennzeichnet, dass** als Emulgator Polyglyceryl-3 Diisostearat enthalten ist.

5. Verwendung der Zubereitung nach einem der vorstehenden Ansprüche, **dadurch gekennzeichnet, dass** nur ein Emulgator enthalten ist.

6. Verwendung der Zubereitung nach einem der vorstehenden Ansprüche, **dadurch gekennzeichnet, dass** Pigmente mit einem Anteil von mehr als 5 Gew.%, insbesondere im Bereich von etwa 15 bis 45 Gew.%, bezogen auf die Gesamtmasse der Zubereitung, enthalten sind.

7. Verwendung der Zubereitung nach einem der vorstehenden Ansprüche, **dadurch gekennzeichnet, dass** als Pigmente nicht-gecoatete Pigmente, insbesondere Eisenoxide, Zinkoxide, Talkum, Titandioxid und/oder deren Kombinationen und insbesondere ZnO enthalten sind.

8. Verwendung der Zubereitung nach einem der vorstehenden Ansprüche, **dadurch gekennzeichnet, dass** keine Lichtschutzfiltersubstanzen und/oder keine gecoateten Pigmente enthalten sind.

## Claims

1. Use of a cosmetic preparation based on an emulsion comprising molecular oxygen obtainable by mixing the emulsion constituents and subsequently gassing the preparation with gaseous oxygen and/or oxygen-containing gases, **characterized in that** the preparation during the gassing has a temperature of at least 40°C, as deodorant and/or antiperspirant.

2. Use of the preparation according to Claim 1, comprising a volume fraction of oxygen at 21°C of more than 10% by volume, based on the total volume of the preparation.

3. Use of the preparation according to one of the preceding claims, **characterized in that** the preparation is a W/O emulsion.

4. Use of the preparation according to one of the preceding claims, **characterized in that** polyglyceryl-3 diisostearate is present as emulsifier.

5. Use of the preparation according to one of the preceding claims, **characterized in that** only one emulsifier is present.

6. Use of the preparation according to one of the preceding claims, **characterized in that** pigments are present with a fraction of more than 5% by weight, in particular in the range from about 15 to 45% by weight, based on the total mass of the preparation.

7. Use of the preparation according to one of the preceding claims, **characterized in that** non-coated pigments, in particular iron oxides, zinc oxides, talc, titanium dioxide and/or combinations thereof and in particular ZnO, are present as pigments.

8. Use of the preparation according to one of the preceding claims, **characterized in that** no light protection filter substances and/or no coated pigments are present.

## Revendications

1. Utilisation d'une préparation cosmétique à base d'une émulsion comprenant de l'oxygène moléculaire, pouvant être obtenue par mélange des composants de l'émulsion et apport subséquent à la préparation d'un gaz consistant en oxygène gazeux et/ou en des gaz contenant de l'oxygène, **caractérisée en ce que** pendant l'apport de gaz la préparation présente une température d'au moins 40 °C, en tant que déodorant et/ou antisudoral.

2. Utilisation de la préparation selon la revendication 1, comprenant une proportion volumique d'oxygène à 21 °C de plus de 10 % en volume, par rapport au volume total de la préparation.

3. Utilisation de la préparation selon l'une quelconque des revendications précédentes, **caractérisée en ce que** la préparation est une émulsion E/H.

4. Utilisation de la préparation selon l'une quelconque des revendications précédentes, **caractérisée en ce que** du Polyglyceryl-3 diisostearate est contenu comme émulsifiant.

5. Utilisation de la préparation selon l'une quelconque des revendications précédentes, **caractérisée en ce qu'**un seul émulsifiant est contenu.

6. Utilisation de la préparation selon l'une quelconque des revendications précédentes, **caractérisée en ce que** des pigments sont contenus en une proportion de plus de 5 % en poids, en particulier dans la plage d'environ 15 à 45 % en poids, par rapport à la masse totale de la préparation.

7. Utilisation de la préparation selon l'une quelconque des revendications précédentes, **caractérisée en ce que** comme pigments sont contenus des pigments non enrobés, en particulier des oxydes de fer, des oxydes de zinc, le talc, le dioxyde de titane et/ou des associations de ceux-ci et en particulier ZnO.

8. Utilisation de à préparation selon l'une quelconque des revendications précédentes, **caractérisée en ce que** des substances de type filtre photoprotecteur et/ou des pigments enrobés ne sont pas contenus.
